# EUROPÄISCHE PATENTSCHRIFT

(11) **EP 2 732 770 B1**
(45) Veröffentlichungstag und Bekanntmachung des Hinweises auf die Patenterteilung: **24.08.2016**
(21) Anmeldenummer: 12192953.3
(22) Anmeldetag: 16.11.2012
(51) Int. Cl.: A61B 17/00

(54) **Nadellose Injektionsvorrichtung**
Needle-free injection device
Dispositif d'injection sans aiguille

(43) Veröffentlichungstag der Anmeldung: 21.05.2014
(73) Patentinhaber: ERBE Elektromedizin GmbH, 72072 Tübingen (DE)
(72) Erfinder: Hagg, Martin, 72827 Wannweil (DE); Kühner, Ralf, 70567 Stuttgart (DE)
(74) Vertreter: Rüger, Barthelt & Abel

(56) Entgegenhaltungen:
- US-A1- 2002 143 323
- US-A1- 2006 149 193

## Beschreibung

Die Erfindung betrifft eine Injektionsvorrichtung zur nadellosen Fluidinjektion, insbesondere zur Unterspritzung bzw. Elevation der Mucosa des Gastrointestinaltrakts oder für ähnliche Anwendungen.

Zur Applikation von Arzneimitteln sind automatische Injektionsgeräte bekannt, die mittels Federspeicher und Nadel arbeiten. Ein Beispiel dafür ist der DE 69533811 T2 zu entnehmen. Das Gerät weist einen Vorratsbehälter für ein Medikament auf, der mit einer Hohlnadel verbunden ist. Diese ist im Ruhezustand in ein Gehäuse zurückgezogen. Eine in dem Gehäuse vorhandene gespannte Feder dient dazu, bei Auslösung die Hohlnadel vorschnellen zu lassen und das Medikament über die Hohlnadel auszustoßen.

Aus der US 2002/0143323 ist eine endoskopische Einrichtung zur gastrointestinalen Epithel-Entfernung bekannt, bei der eine Sonde mit einer Flüssigkeit versorgt wird. Diese wird der Sonde aus einem Vorratsbehälter zugeführt, der mit unter Druck stehendem Gas aus einer Gasflasche beaufschlagbar ist.

Außerdem ist aus der US 2006/0149193 A1 eine Beinrichtung mit einer Sonde und einem Fluidapplikator bekannt, der eine Fluidaustrittsöffnung zur nadellosen Injektion eines Fluids in ein biologisches Gewebe und eine zu der Fluidaustrittsöffnung führende Fluidleitung aufweist. Eine zugeordnete Fluidliefereinrichtung weist eine Antriebseinrichtung auf und ist mit einem druckspeichernden Druckbehälter als Energiespeicher verbindbar. Zu der Fluidliefereinrichtung gehört eine Expansionskammer, die eine bewegliche Wandfläche aufweist, die das zu applizierende Fluid umschließt und mit einem Druckfluid beaufschlagbar ist.

Darüberhinaus sind Geräte bekannt, die zur nadellosen Injektion eines Fluids unter die Mucosa dienen. Zum Beispiel offenbart die US 2009/0157114 A1 ein Endoskop mit einer Sonde zur nadellosen Unterspritzung der Mucosa. Die Sonde stößt dazu einen Strahl Natriumchloridlösung aus, der aufgrund seines geringen Querschnitts und seines Drucks (bzw. Impulses) in das Gewebe eindringt. Zur Förderung der Natriumchloridlösung und zur Erzeugung des entsprechenden Drucks kann eine Pumpeinheit oder gegebenenfalls ein kraftverstärkender Hebel vorgesehen sein.

Die Anwendung dieses Prinzips setzt ein speisendes Gerät mit einer entsprechenden Pumpeinheit voraus. Wird zur Druckerzeugung ein kraftverstärkender Hebel, gegebenenfalls in Verbindung mit einer entsprechenden druckfesten Spritze, genutzt, ist die permanente Druckerzeugung durch Ausübung einer Handkraft z.B. durch einen Assistenten umständlich.

Davon ausgehend ist es Aufgabe der Erfindung, eine Injektionsvorrichtung zu schaffen, die leicht und komfortabel zu handhaben ist und geringen technischen Aufwand erfordert.

Diese Aufgabe wird mit der erfindungsgemäßen Injektionsvorrichtung gelöst, die insbesondere zur nadellosen Unterspritzung bzw. Elevation der Mucosa des Gastrointestinaltrakts eingerichtet, jedoch auch bei anderen vergleichbaren Aufgaben genutzt werden kann. Die Injektionsvorrichtung dient dazu, mittels eines Fluidapplikators einen Fluidstrahl zu erzeugen, der ohne Nutzung einer Hohlnadel in das biologische Gewebe eindringt und dort ein Flüssigkeitsdepot erzeugt. Der Fluidapplikator ist vorzugsweise als flexible dünne Sonde ausgebildet, die durch einen Schlauch, ein Endoskop oder andere geeignete Mittel hindurch in eine Körperhöhle eingeführt werden kann, um dort die gewünschte nadellose Injektion vorzunehmen. Für die offenchirurgische Anwendung kann der Fluidapplikator jedoch auch als Handinstrument mit einem Griff und einer Düse ausgebildet sein, über die der Anwender das Ausstoßen eines Fluidstrahls veranlasst. Unabhängig davon, ob der Fluidapplikator als flexible Sonde oder als Handinstrument ausgebildet ist, kann die zum Ausstoßen des Fluidstrahls vorgesehene Düse einen im Wesentlichen zylindrischen scharfen Strahl oder auch andere beispielsweise zur Paremchymauflösung geeignete Strahlformen erzeugend (Kegelstrahl, Fächerstrahl, Hohlstrahl und dergleichen) ausgebildet sein. Es ist auch möglich, den von der erfindungsgemäßen Vorrichtung erzeugte Fluidstrahl zur Dissektion von Gewebe zu verwenden.

Die zur Speisung der Fluidaustrittsöffnung führende Fluidleitung ist mit einer Vorratskammer verbunden, die zu einer Fluidliefereinrichtung gehört. Das Fluid ist vorzugsweise physiologische Kochsalzlösung. Die Vorratskammer weist zumindest eine erste bewegliche Wandfläche auf, die mit einer von einer Antriebseinrichtung erzeugten Kraft beaufschlagbar ist. Vorzugsweise ist diese Kraft zeitlich im Wesentlichen konstant, so dass an der Fluidaustrittsöffnung das Fluid mit im Wesentlichen konstanter Geschwindigkeit und unter im Wesentlichen konstantem Druck austreten kann.

Die Antriebseinrichtung weist einen Energiespeicher auf. Damit kann sie unabhängig von stationären Geräten gehandhabt werden, was eine hohe Flexibilität beim Einsatz am Patienten mit sich bringt. Der Energiespeicher ersetzt auch Hilfspersonen, die sonst den zur nadellosen Injektion erforderlichen hohen Fluiddruck von Hand aufbringen müssten. Vorzugsweise steht das Fluid unter einem Druck von 20 bar bis 40 bar, beispielsweise hat es einen Druck von im Wesentlichen konstant 30 bar.

Bei einer bevorzugten Ausführungsform ist der Energiespeicher als Druckquelle ausgebildet. Die Druckquelle wird durch ein Vorratsvolumen mit einem unter Druck stehenden kompressiblen Fluid verstanden. Das Fluid kann ganz oder teilweise in gasförmigem oder in flüssigem Zustand vorliegen. Vorzugsweise wird als Fluid ein bei Zimmertemperatur und Speicherdruck in flüssiger Form vorliegendes Fluid wie Kohlendioxid (CO₂), Stickstoffoxid (Distickstoffmonoxid o.ä.) verwendet. Bei der Expansion verdampft es ganz oder teilweise. Die Anwendung anderer (nichtsiedender) Fluide (Stickstoff, Argon oder dergleichen) ist möglich.

Die Antriebseinrichtung umfasst zur Expansion des treibenden Fluids vorzugsweise eine Expansionskammer mit einer zweiten beweglichen Wandfläche, wobei die Expansionskammer mit der Druckquelle verbunden oder verbindbar ist. Sobald die Druckquelle mit der Expansionskammer verbunden ist, ist die Injektionsvorrichtung betriebsbereit. Der von dem treibenden Fluid auf die zweite bewegliche Wandfläche ausgeübte Druck erzeugt eine Kraft, mit der die erste bewegliche Wandfläche der Vorratskammer beaufschlagbar ist. Ist in der Druckquelle wenigstens ein Teil des treibenden Fluids in flüssiger Phase vorhanden, kann in der Expansionskammer während der Expansion, d.h. während des Ausstoßens des Fluid, ein konstanter Arbeitsdruck aufrechterhalten werden. Entsprechend wird das Fluid mit konstantem Druck beaufschlagt.

Als Gasdruckspeicher kommt vorzugsweise eine CO₂-Kapsel, eine N₂O-Kapsel oder eine andere gasgefüllte Kapsel mit einer gasdichten Versiegelung zur Anwendung. Der Kapsel kann eine Aufnahme mit einer Durchstecheinrichtung für die Entsiegelung der Kapsel zugeordnet sein. Sobald die Kapsel in eine entsprechende Aufnahme eingesetzt und mittels der Durchstecheinrichtung aktiviert ist, ist die Injektionsvorrichtung betriebsbereit. Die erste und die zweite bewegliche Wandfläche weisen unterschiedliche Flächeninhalte auf. Auf diese Weise wird eine Druckanpassung bewirkt und beispielsweise mit einer bis zu 60 bar aufweisenden CO₂-Kapsel ein konstanter Ejektionsdruck von zum Beispiel 30 bar erreicht. Das Flächenverhältnis der Wandflächen zueinander ist in diesem Fall 1:2. Andere Wandflächenverhältnisse können vorgesehen werden. Damit ist sowohl eine Druckerhöhung als auch eine Druckverminderung erzielbar.

Vorzugsweise sind die beiden Flächen an voneinander entfernt liegenden Kolben angeordnet, zwischen denen eine Ventilationsöffnung vorgesehen ist. Auf diese Weise kann an dem Kolben der Expansionskammer austretendes Leckgas entweichen, ohne in die Vorratskammer einzudringen. Schädigungen des Patienten durch durchschlagende Gase sind hiermit ausgeschlossen.

Vorzugsweise ist zwischen der Düse und der Vorratskammer ein Ventil vorgesehen. Es kann sich hier um ein Sperrventil handeln, über das der Fluidstrom freigegeben oder abgesperrt werden kann. Bei einer komfortablen Ausführungsform kann das Ventil auch als Drosselventil ausgebildet sein. Es gestattet dem Anwender die Regulierung der Stärke des austretenden Fluidstrahls.

Typischerweise ist das Ventil in der Nähe der Vorratskammer angeordnet. Bei Inbetriebnahme der Injektionsvorrichtung muss die Fluidleitung entlüftet werden. Dies geschieht durch probeweises Betätigen des Ventils bis die gesamte Fluidleitung mit Fluid gefüllt ist. Alternativ kann die Fluidaustrittsöffnung auch mit einem Sterilitätsverschluss versehen und die Fluidleitung mit Fluid vorgefüllt sein. Dies erleichtert die Handhabung weiter.

Jede der vorstehend beschriebenen Injektionsvorrichtungen kann ganz oder teilweise als Einweggerät ausgebildet sein. Ein Gehäuse, das die Vorratskammer und die Antriebseinrichtung enthält, kann zum Beispiel steril aus Kunststoff bereitgestellt sein, wobei die Vorratskammer mit Natriumchloridlösung oder einem anderen Fluid gefüllt sein kann. Bei der erfindungsgemäßen druckspeicherbetätigten Ausführungsform kann eine sterile Packung sowohl die Injektionsvorrichtung wie auch den Druckspeicher bspw. in Gestalt einer CO₂-Kapsel oder anderen Kapsel enthalten. Die Kapsel wird zur Aktivierung in eine entsprechende Aufnahme der Injektionsvorrichtung eingesetzt und angestochen. Es ist auch möglich nichtsterile Speicherkapseln (z.B. NO₂, CO₂ oder dergleichen) aus dem Konsumgüterbereich zu nutzen.

Weitere Einzelheiten von Ausführungsformen der Erfindung sind Gegenstand der Zeichnung, der Beschreibung oder von Unteransprüchen.

In der Zeichnung sind in unterschiedlichen Figuren Ausführungsformen der erfindungsgemäßen Injektionsvorrichtung schematisch und beispielhaft veranschaulicht. Es zeigen:
Figur 1 eine erfindungsgemäße Injektionsvorrichtung, in schematisierter Gesamtansicht,
Figur 2 einen Teil einer alternativen Ausführungsform einer erfindungsgemäßen Injektionsvorrichtung,
Figur 3 eine Fluidliefereinrichtung einer Injektionsvorrichtung, in einer ersten Ausführungsform, und
Figur 4 eine Fluidliefereinrichtung für eine Injektionsvorrichtung in abgewandelter Ausführungsform.

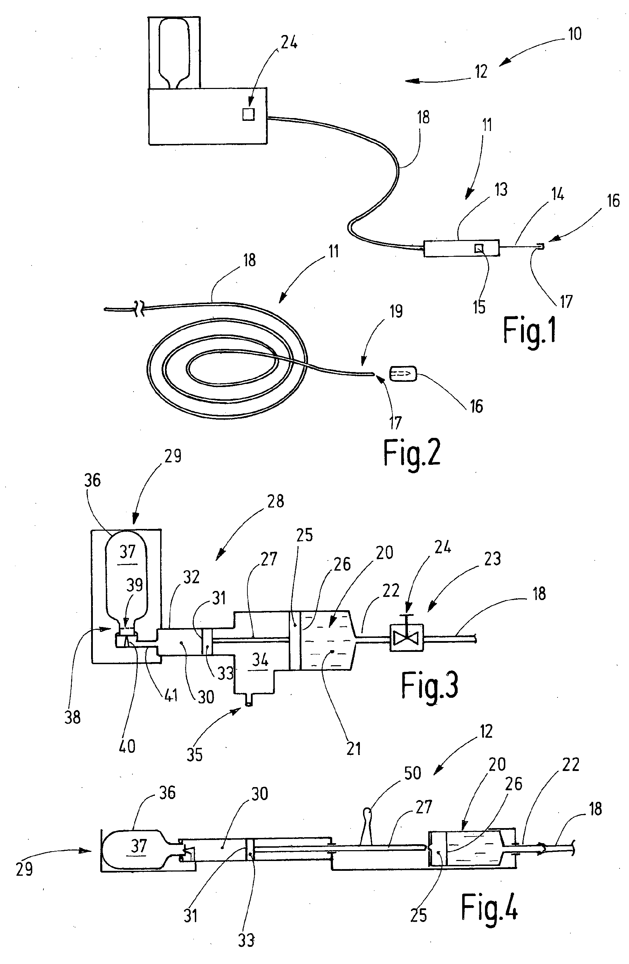

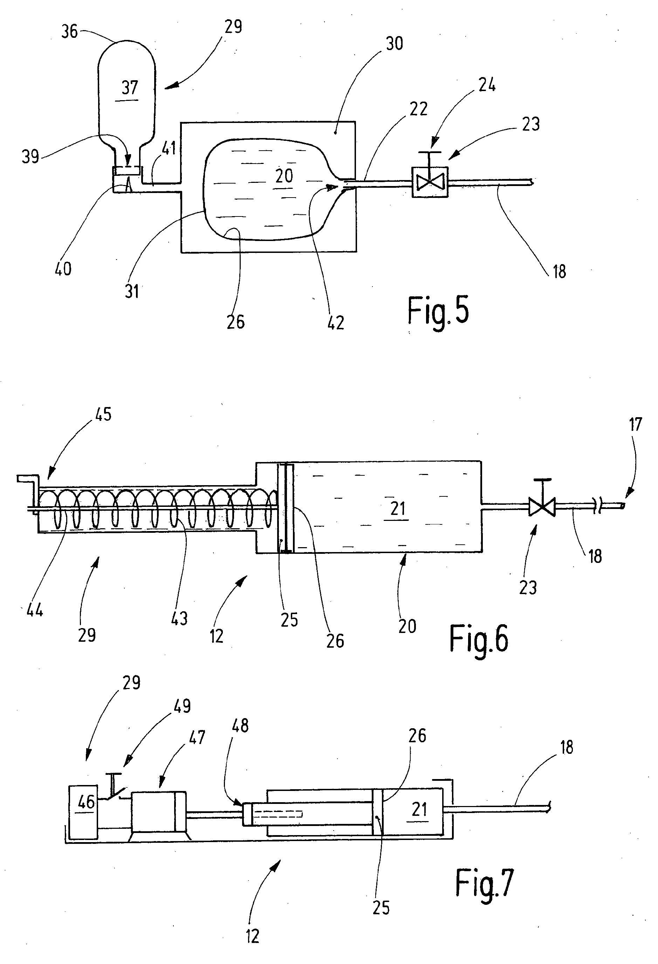

Die in Figur 1 veranschaulichte Injektionsvorrichtung 10 umfasst einen Fluidapplikator 11 und eine Fluidliefereinrichtung 12 zur Speisung des Fluidapplikators 11 mit einem Fluid. Das Fluid ist vorzugsweise eine physiologische Kochsalzlösung (NaCl-Lösung), die von dem Fluidapplikator 11 in einer geeigneten Strahlform ausgestoßen wird. Vorzugsweise wir ein scharfer laminarer, nadelartiger Strahl bevorzugt. Es können jedoch auch Anwendungsfälle vorgesehen werden, bei denen eine andere Strahlform gewünscht und realisiert wird.

Der Fluidapplikator 11 kann, wie in Figur 1 schematisch veranschaulicht, zur offenchirurgischen Applikation vorgesehen sein und entsprechend einen Handgriff 13 und einen sich von diesem weg erstreckenden Düsenschaft 14 aufweisen. An dem Handgriff 13 kann ein Betätigungsorgan, bspw. in Gestalt einer Taste 15, vorgesehen sein, über die ein die Speisung des Düsenschafts 14 freigebendes oder sperrendes Ventil oder auch ein Regulierventil betätigbar ist. An dem distalen Ende des Düsenschafts 14 kann eine hier durch eine Schutzkappe 16 verschlossene Fluidaustrittsöffnung 17 angebracht sein, die einen Fluidstrahl der gewünschten Form austreten lassen kann. Die Fluidaustrittsöffnung 17 wird vorzugsweise durch eine Düse gebildet. Zu dem Fluidapplikator 11 gehört ferner eine Fluidleitung 18, die den Fluidapplikator 11 mit der Fluidliefereinrichtung 12 verbindet. Die Fluidleitung 18 führt das Fluid durch den Handgriff 13 hindurch letztendlich zu dem Düsenschaft 14 und seiner Düse.

Während Figur 1 einen Fluidapplikator 11 zur offenchirurgischen Anwendung veranschaulicht, ist in Figur 2 ein Fluidapplikator 11 zur endoskopischen Anwendung veranschaulicht. Er umfasst im Wesentlichen die Fluidleitung 18, die hier als dünner flexibler Kunststoffschlauch ausgebildet ist, der durch ein Lumen eines Endoskops geschoben werden kann. An seinem distalen Ende 19 ist die Fluidaustrittsöffnung 17 angeordnet. Falls erforderlich kann die Austrittsöffnung durch eine Düse festgelegt sein.

Die Fluidliefereinrichtung 12 dient zur Versorgung des Fluidapplikators 11 mit unter einem im Wesentlichen konstanten Druck stehenden Fluid. Sowohl der Fluidapplikator 11 nach Figur 1 wie auch der Fluidapplikator 11 nach Figur 2 kann von jeder der nachstehend beschriebenen Fluidliefereinrichtungen 12 mit Fluid versorgt werden.

Wie Figur 3 zeigt, weist die Fluidliefereinrichtung 12 eine Vorratskammer 20 auf, die vollständig mit einem geeigneten Fluid 21 gefüllt ist. Die Vorratskammer 20 weist einen Ausgang 22 auf, an dem über ein Ventil 23 die Fluidleitung 18 angeschlossen ist. Das Ventil 23 kann ein schaltendes Ventil sein, das nur die beiden Stellungen "offen" und "geschlossen" einnehmen kann. Alternativ kann es sich um ein Drosselventil handeln, mit dem verschiedene Durchflusswiderstände und somit verschiedene Durchflussmengen einstellbar sind. Das Ventil kann ein Bedienorgan bzw. Betätigungsorgang 24 aufweisen, das an einem Gehäuse der Fluidliefereinrichtung 12 angebracht ist.

Die Vorratskammer 20 wird durch einen Aufnahmeraum, der bspw. die Form eines Zylinders aufweist, gebildet, der an einer Seite durch einen beweglichen Kolben 25 verschlossen ist. Der Kolben schließt die Vorratskammer 20 mit einer Wandfläche 26 ab, die bezüglich des übrigen Zylinders beweglich ist, indem der Kolben 25 gegen den Zylinder abgedichtet verschiebbar ist.

Der Kolben 25 ist über ein Kraftübertragungsmittel, wie bspw. einen Zapfen, einen Stößel, eine Stange 27 oder dergleichen, mit einer Antriebseinrichtung 28 verbunden, die einen Energiespeicher 29 enthält. Die Antriebseinrichtung 28 umfasst im vorliegenden Ausführungsbeispiel außerdem eine Expansionskammer 30, die wenigstens eine bewegliche Wandfläche 31 hat. Das Verhältnis der Wandflächen 26, 31 zueinander ist vorzugsweise verschieden von Eins. Die bewegliche Wandfläche 31 kann durch die Stirnfläche eines in einem Zylinder 32 abgedichtet verschiebbar gelagerten Kolben 33 gebildet sein. Dieser ist über die Stange 27 oder ein sonstiges Kraftübertragungsmittel mit dem Kolben 25 verbunden. Die Stange 27 erstreckt sich durch einen Zwischenraum 34, der ganz oder teilweise von dem Gehäuse umschlossen ist, das die Vorratskammer 20 und die Expansionskammer 30 umschließt. Der Zwischenraum 34 ist vorzugsweise zur Atmosphäre belüftet, wozu mindestens eine Belüftungsöffnung 35 vorgesehen ist.

Der Energiespeicher 29 bildet eine Druckquelle 36, beispielsweise in Gestalt einer CO₂-Kapsel 37. Diese wird durch einen druckfesten Behälter gebildet, der an einem halsartigen Ende 38 mit einer Versiegelung 39 versehen ist. Die Versiegelung 39 kann zur Freisetzung des in der Druckquelle bzw. dem Druckbehälter 36 vorhandenen Gases mittels einer Aufstechnadel 40 durchstochen werden, die Teil einer Behälteraufnahme ist. Die Behälteraufnahme weist Dichtmittel auf, um den Druckbehälter 36 an seinem Hals bzw. Ende 38 abgedichtet aufzunehmen. Die Aufstechnadel 40 durchsticht die Versiegelung 39, wodurch das somit freigesetzte unter Druck stehende Gas über einen Kanal 41 oder auch unmittelbar (Fig. 4) in die Expansionskammer 30 geleitet werden kann.

Die insoweit beschriebene Injektionsvorrichtung 10 arbeitet wie folgt:

Es wird davon ausgegangen, dass die Fluidliefereinrichtung 12 mit gefüllter Vorratskammer 20 bereitgestellt wird. Die Kolben 25, 33 befinden sich somit in Figur 3 in linker Position. Das Ventil 23 ist geschlossen und die Fluidleitung 18 zunächst leer.

Zur Aktivierung wird der Druckbehälter 36 in seine Aufnahme eingesetzt, wobei die Versiegelung 39 durchstoßen wird. Alternativ wird ein bereits in der Aufnahme vorhandener vorinstallierter Druckbehälter mittels eines geeigneten Mechanismus angestochen, indem er gegen die Aufstechnadel 40 bewegt oder indem die Aufstechnadel bewegt wird. Der Mechanismus kann z.B. ein handhebelbetätigter Exzenter sein, an dem sich das dem Hals gegenüberliegende Ende 38 des Druckbehälters 36 abstützt. Andere Mechanismen, wie Kniehebelanordnungen oder dergleichen können vorgesehen sein.

Handelt es sich bei dem Druckbehälter 36 um eine handelsübliche CO₂-Kapsel mit einer Füllung von z.B. 12g C0₂, wirkt unmittelbar ein Druck von 55 bar bis 60 bar auf die Wandfläche 31. Die entsprechende in Figur 3 nach rechts gerichtete Kraft wird über die Stange 27 auf den Kolben 25 übertragen. Infolge des Durchmesserverhältnisses der Kolben 33, 25 zueinander, kann ein gewünschter Fluiddruck von beispielsweise 30 bar hervorgerufen werden. In diesem Fall hat die Wandfläche 26 den doppelten Flächeninhalt wie die Wandfläche 31.

Der Anwender betätigt nun das Ventil 23, um es zu öffnen und die Fluidleitung 18 bis zur Fluidaustrittsöffnung 17 mit Fluid zu füllen. Wenn eine Schutzkappe 16 vorhanden ist, nimmt er diese zuvor ab. Die Injektionsvorrichtung 10 ist nun betriebsbereit. Je nach Bauform des Fluidapplikators 11 kann diese durch entsprechende Handhabung des Handgriffs 13 und bedarfsweise Betätigung der Taste 15 (falls vorhanden) benutzt werden. Tritt aus der Fluidaustrittsöffnung 17 ein scharfer Fluidstrahl aus, kann dieser in ein biologisches Gewebe eindringen und dort zum Beispiel zum Unterspritzen der Mucosa genutzt werden. Andere Anwendungen zum Einspritzen in und Unterspritzen von Geweben sind möglich. Der Anwender kann bspw. mit dem austretenden Strahl auch Organgewebe auflösen. Dazu kann die Fluidaustrittsöffnung 17 auch so gestaltet werden, dass anderen Strahlformen bspw. Fächerstrahlen oder dergleichen erzeugt werden.

Bei Anwendung des Fluidapplikators 11 nach Figur 2 wird dieser zum Beispiel durch ein Endoskop in eine Körperhöhle, bspw. den Gastrointestinaltrakt, geführt, um dort mit dem aus der Fluidaustrittsöffnung 17 austretendem Fluidstrahl Unterspritzungen bspw. der Mucosa vorzunehmen.

Der Fluidvorrat kann auf 200 ml bis 300 ml bemessen sein. Ein solcher Fluidvorrat kann mit einer einzigen CO₂-Kapsel 37 (z.B. 12 g) mit nahezu konstantem Druck von 30 bar ausgestoßen werden. Somit ergeben sich für den Anwender verlässliche und konstante Operationsbedingungen. Die Injektionsvorrichtung 10 ist dabei insgesamt sehr einfach zu handhaben und unabhängig von Fremdenergie oder von der Geschicklichkeit eines Assistenten einsetzbar.

Es sind Abwandlungen möglich, die nachfolgend exemplarisch erläutert werden und die insbesondere die Fluidliefereinrichtung 12 betreffen. Beispielsweise kann der Druckbehälter 36 sowohl wie in Figur 3 dargestellt, in einem Winkel zu der Bewegungsrichtung des Kolbens 33 wie auch koaxial zu diesem angeordnet sein, wie Figur 4 veranschaulicht. Damit kann eine kompakte schlanke Bauform erreicht werden. Außerdem ist es sowohl möglich, die Injektionsvorrichtung 10 insgesamt oder lediglich die Fluidliefereinrichtung 12 als Einmalgerät oder als mehrfach verwendbare Vorrichtung auszubilden. Figur 4 veranschaulicht dies an einem Beispiel, bei dem die Vorratskammer 20 und ihr Kolben 25 als gesondert handhabbare Baugruppe nach Art einer druckfesten Spritze ausgebildet sind. Diese wird in eine Fassung des Gehäuses eingelegt, wobei die Stange 27 gegen den Kolben 25 drückt. Sobald der Druckbehälter 36, z.B. eine CO₂-Kapsel 37 in ihre entsprechende Fassung eingesetzt ist, ist die Kammer 30 druckbeaufschlagt, womit die Stange 27 mit im Wesentlichen konstanter Kraft gegen den Kolben 25 drückt. Über die an den Ausgang 22 angeschlossene Fluidleitung 18 kann nun wie vorbeschrieben gearbeitet werden. Das Ventil 23 kann in dem Handgriff 13 oder an anderer Stelle der Fluidleitung 18 angeordnet sein.

Ist die Vorratskammer 20 leer, kann sie durch eine sterile, volle ersetzt werden. Ebenso kann die CO₂-Kapsel 37 entsorgt werden, womit die Expansionskammer 30 zunächst drucklos wird. Über ein geeignetes Mittel 50 kann der Kolben 25 in seine linke Ruheposition zurückgeschoben werden. In diesem Zustand können eine neue Vorratskammer 20, d.h. eine neue Spritze und eine neue CO₂-Kapsel 37 in das Gehäuse eingesetzt werden, womit die Fluidliefereinrichtung 12 wiederum betriebsbereit ist. Bei solchen wiederverwendbaren Vorrichtungen kann an die Expansionskammer 30 auch ein Restdruckentleerungsventil angeschlossen sein, um vorhandenes restliches Treibfluid gefahrlos ablassen zu können.

Die erfindungsgemäße Injektionsvorrichtung 10 weist einen Fluidapplikator 11 auf, der von einer Fluidliefereinrichtung 12 gespeist wird, die einen Energiespeicher 29 enthält. Dieser ist als Druckbehälter 36 ausgebildet. Der von diesem Druckbehälter 36 ausgehende Gasdruck wirkt indirekt auf einen mit der Flüssigkeit bzw. dem Fluid (z.B. isotonischer NaCL-Lösung) vorgefüllten Behälter (Figur 3, 4). Die auszustoßende Flüssigkeit wird somit unter konstanten Druck gesetzt. Mit einem Ventil 23 kann der Anwender den Ausstoß der gewünschten Flüssigkeitsmenge mit konstanter Intensität veranlassen. Das Sperrventil 23 kann auch ein Drosselventil sein, womit die Intensität des Effekts durch den Anwender variiert werden kann. Die Erfindung bietet die Basis, eine sehr kostengünstig herstellbare immer verfügbare Injektionsvorrichtung zu realisieren. Der Anwender muss lediglich das System entsichern, z.B. indem er den Druckbehälter 36 ansticht, um dann über die Fluidleitung 18, d.h. den Fluidapplikator 11, unmittelbar Flüssigkeit definiert bzw. dosiert injizieren zu können. Die Handhabung sowohl des Entsicherns als auch die Betätigung des Sperr- oder Drosselventils 23 ist einfach und intuitiv möglich. Es ist auch möglich, die gesamte Injektionsvorrichtung 10 als Einmalsystem auszubilden, und dem Fluidapplikator 11 bis zu seiner Fluidaustrittsöffnung hin vorzufüllen. Ein steriler Verschluss am distalen Ende des Fluidapplikators 12 kann durch eine Schutzkappe 16 gebildet werden.

### Bezugszeichenliste:

- 10: Injektionsvorrichtung
- 11: Fluidapplikator
- 12: Fluidliefereinrichtung
- 13: Handgriff
- 14: Düsenschaft
- 15: Taste
- 16: Schutzkappe
- 17: Fluidaustrittsöffnung
- 18: Fluidleitung
- 19: distales Ende
- 20: Vorratskammer
- 21: Fluid
- 22: Ausgang
- 23: Ventil
- 24: Betätigungsorgan
- 25: Kolben
- 26: erste bewegliche Wandfläche
- 27: Stange
- 28: Antriebseinrichtung
- 29: Energiespeicher
- 30: Expansionskammer
- 31: zweite bewegliche Wandfläche
- 32: Zylinder
- 33: Kolben
- 34: Zwischenraum
- 35: Belüftungsöffnung
- 36: Druckbehälter, Druckquelle
- 37: CO₂-Kapsel
- 38: Ende
- 39: Versiegelung
- 40: Aufstechnadel
- 41: Kanal
- 50: Mittel zur Handhabung

## Patentansprüche

1. Injektionsvorrichtung (10) mit:
einem Fluidapplikator (11), der eine Fluidaustrittsöffnung (17) zur nadellosen Injektion eines Fluids in ein biologisches Gewebe und eine zu der Fluidaustrittsöffnung (17) führende Fluidleitung (18) aufweist,
die den Fluidapplikator (11) mit einer Fluidliefereinrichtung (12) verbindet, die eine Antriebseinrichtung (28) und eine Vorratskammer (20) zur Aufnahme des Fluids aufweist, wobei die Antriebseinrichtung (28) einen druckspeichernden Druckbehälter (36) als Energiespeicher (29) und eine Expansionskammer (30) aufweist, die eine zweite bewegliche Wandfläche (31) aufweist, die von dem Energiespeicher (29) mit einem Druckfluid beaufschlagbar ist und somit eine Kraft erzeugt,
wobei die Vorratskammer (20) wenigstens eine erste bewegliche Wandfläche (26) aufweist, die mit der von der Antriebseinrichtung (28) erzeugten Kraft beaufschlagbar ist,
**dadurch gekennzeichnet, dass**
die erste bewegliche Wandfläche (26) und die zweite bewegliche Wandfläche (31) unterschiedliche Flächeninhalte aufweisen.

2. Injektionsvorrichtung nach Anspruch 1, **dadurch gekennzeichnet, dass** der Druckbehälter (36) eine CO₂-Kapsel (37) mit einer gasdichten Versiegelung (39) ist.

3. Injektionsvorrichtung nach Anspruch 1, **dadurch gekennzeichnet, dass** dem Energiespeicher (29) eine Wechselaufnahme für den Druckbehälter (36) und eine Durchstecheinrichtung (40) für eine Versiegelung (39) des Druckbehälters (36) zugeordnet ist.

4. Injektionsvorrichtung nach Anspruch 1, **dadurch gekennzeichnet, dass** zwischen der ersten Wandfläche (26) und der zweiten Wandfläche (31) ein belüfteter Raum (34) angeordnet ist.

5. Injektionsvorrichtung nach einem der vorigen Ansprüche, **dadurch gekennzeichnet, dass** zwischen der Vorratskammer (20) und der Fluidaustrittsöffnung (17) ein Ventil (23) angeordnet ist.

6. Injektionsvorrichtung nach Anspruch 6, **dadurch gekennzeichnet, dass** das Ventil (23) ein Sperrventil ist.

7. Injektionsvorrichtung nach Anspruch 6, **dadurch gekennzeichnet, dass** das Ventil (23) ein Drosselventil ist.

8. Injektionsvorrichtung nach einem der vorigen Ansprüche, **dadurch gekennzeichnet, dass** die Fluidaustrittsöffnung (17) mit einer Schutzkappe (16) versehen ist.

## Claims

1. Injection device (10) having:
a fluid applicator (11) which has a fluid outlet opening (17) for the needle-free injection of a fluid into a biological tissue and a fluid line (18) leading to the fluid outlet opening (17),
said fluid line connecting the fluid applicator (11) to a fluid delivery device (12) which has a drive device (28) and a storage chamber (20) for receiving the fluid,
wherein the drive device (28) has a pressure accumulating pressure vessel (36) as an energy store (29) and an expansion chamber (30) which has a second moveable wall surface (31) which is able to be supplied with a pressurised fluid by the energy store (29) and thus generates a force,
wherein the storage chamber (20) has at least one first moveable wall surface (26) which is able to be supplied with the force generated by the drive device (28),
**characterised in that**
the first moveable wall surface (26) and the second moveable wall surface (31) have different surface areas.

2. Injection device according to claim 1, **characterised in that** the pressure vessel (36) is a CO₂ capsule (37) having a gas-tight seal (39).

3. Injection device according to claim 1, **characterised in that** an interchangeable receiver for the pressure vessel (36) and a puncturing means (40) for a seal (39) of the pressure vessel (36) are allocated to the energy store (29).

4. Injection device according to claim 1, **characterised in that** a ventilated space (34) is arranged between the first wall surface (26) and the second wall surface (31).

5. Injection device according to one of the preceding claims, **characterised in that** a valve (23) is arranged between the storage chamber (20) and the fluid outlet opening (17).

6. Injection device according to claim 6, **characterised in that** the valve (23) is a stop valve.

7. Injection device according to claim 6, **characterised in that** the valve (23) is a throttle valve.

8. Injection device according to one of the preceding claims, **characterised in that** the fluid outlet opening (17) is provided with a protective cap (16).

## Revendications

1. Dispositif d'injection (10), comprenant :
un applicateur de fluide (11) qui présente un orifice de sortie de fluide (17), destiné à l'injection sans aiguille d'un fluide dans un tissu biologique, et un conduit de fluide (18) menant à l'orifice de sortie de fluide (17),
qui relie l'applicateur de fluide (11) à un dispositif d'alimentation en fluide (12) qui présente un dispositif d'entraînement (28) et une chambre-réservoir (20) destinée à recevoir le fluide, sachant que le dispositif d'entraînement (28) présente un récipient sous pression (36) à accumulation de pression, en tant qu'accumulateur d'énergie (29), et une chambre à détente (30) qui présente une deuxième surface de paroi (31) mobile qui peut être sollicitée avec un fluide sous pression par l'accumulateur d'énergie (29) et produit ainsi une force,
la chambre-réservoir (20) présentant au moins une première surface de paroi (26) mobile qui peut être sollicitée par la force produite par le dispositif d'entraînement (28),
**caractérisé en ce que**
la première surface de paroi (26) mobile et la deuxième surface de paroi (31) mobile présentent des superficies différentes.

2. Dispositif d'injection selon la revendication 1, **caractérisé en ce que** le récipient sous pression (36) est une capsule de CO₂ (37) avec un scellement (39) étanche au gaz.

3. Dispositif d'injection selon la revendication 1, **caractérisé en ce que** sont associés à l'accumulateur d'énergie (29), un logement interchangeable pour le récipient sous pression (36) et un dispositif de perforation (40) pour un scellement (39) du récipient sous pression (36).

4. Dispositif d'injection selon la revendication 1, **caractérisé en ce qu'**un espace ventilé (34) est disposé entre la première surface de paroi (26) et la deuxième surface de paroi (31).

5. Dispositif d'injection selon l'une des revendications précédentes, **caractérisé en ce qu'**une valve (23) est disposée entre la chambre-réservoir (20) et l'orifice de sortie de fluide (17).

6. Dispositif d'injection selon la revendication 6, **caractérisé en ce que** la valve (23) est une valve d'arrêt.

7. Dispositif d'injection selon la revendication 6, **caractérisé en ce que** la valve (23) est une valve d'étranglement.

8. Dispositif d'injection selon l'une des revendications précédentes, **caractérisé en ce que** l'orifice de sortie de fluide (17) est pourvu d'un capuchon de protection (16).
